# EUROPEAN PATENT APPLICATION

(11) **EP 4 740 898 A1**
(43) Date of publication of application: **13.05.2026**
(21) Application number: 24836261.8
(22) Date of filing: 28.06.2024
(51) Int. Cl.: A61C 5/30, A61C 9/00, A61C 19/04, A61B 5/00, A61B 6/51, A61B 6/03, A61B 6/46, G06F 17/00, G06F 30/20

(54) **ELECTRONIC DEVICE, METHOD, AND RECORDING MEDIUM FOR GENERATING CERVICAL PROSTHESIS**

(30) Priority: 06.07.2023 KR 20230087606; 25.06.2024 KR 20240082400
(71) Applicant: Medit Corp., Seoul 07207 (KR)
(72) Inventor: KOO, Minkyo, Seoul 07207 (KR)
(74) Representative: MFG Patentanwälte Meyer-Wildhagen Meggle-Freund Gerhard PartG mbB
(86) International application number: PCT/KR2024/009049
(87) International publication number: WO 2025/009813

(57) **Abstract**

The present disclosure relates to an electronic device, a method, and a recording medium for generating cervical prosthesis. An electronic device according to an embodiment of the present disclosure may include: at least one processor; and at least one memory configured to store instructions to be executed by the at least one processor, wherein the at least one processor is configured to, when the instructions are executed by the at least one processor: acquire three-dimensional data of a subject's oral cavity, the three-dimensional data including three-dimensional coordinate values; based on the three-dimensional data, generate a three-dimensional image model; based on the three-dimensional image model, determine a first line included in a cervical prosthesis for a specific tooth of the subject; based on the first line, determine a second line included in a guide portion configured to assist insertion of the cervical prosthesis; based on the first line, generate information representing the cervical prosthesis; and based on the second line, generate information representing the guide portion.

## Description

### TECHNICAL FIELD

The present disclosure relates to a technology for generating a cervical prosthesis and, more particularly, to a technology for generating a cervical prosthesis that can be inserted into a worn cervical portion of a subject.

### BACKGROUND ART

A cervical inlay procedure is a process of inserting a prosthesis into a worn cervical portion (the boundary area between the tooth and gingiva). Conventional cervical inlay procedures necessarily involve a process of manually inserting a prefabricated prosthesis into the worn cervical portion. However, manually inserting the prosthesis near a gingival region was an extremely inconvenient task for dentists. Therefore, there has been a need for a technique that facilitates the attachment of a prosthesis to a worn cervical portion.

### DISCLOSURE

### TECHNICAL PROBLEM

A technical problem to be solved by an embodiment of the present disclosure is to facilitate attachment of a prosthesis to a worn cervical portion.

### TECHNICAL SOLUTION

Another technical problem to be solved by an embodiment of the present disclosure is to manufacture a prosthesis having an appropriate shape to be inserted into a worn cervical portion.

Another technical problem to be solved by an embodiment of the present disclosure is to simplify a process of generating a cervical prosthesis using a 3D printer and CAD.

Technical problems of the present disclosure are not limited to the above-described technical problems, and other technical problems not described herein will be clearly understood by those skilled in the art of the present disclosure from the following description.

### TECHNICAL SOLUTION

An electronic device according to an embodiment of the present disclosure may include: at least one processor; and at least one memory configured to store instructions to be executed by the at least one processor, wherein the at least one processor is configured to, when the instructions are executed by the at least one processor: acquire three-dimensional data of a subject's oral cavity, the three-dimensional data including three-dimensional coordinate values; based on the three-dimensional data, generate a three-dimensional image model; based on the three-dimensional image model, determine a first line included in a cervical prosthesis for a specific tooth of the subject; based on the first line, determine a second line included in a guide portion configured to assist insertion of the cervical prosthesis; based on the first line, generate information representing the cervical prosthesis; and based on the second line, generate information representing the guide portion.

In an embodiment, an internal area of a closed curve obtained by projecting the second line in a specific direction, which is an insertion direction of the cervical prosthesis, onto a plane perpendicular to the specific direction, may be larger than an internal area of a closed curve obtained by projecting the first line in the specific direction onto the plane perpendicular to the specific direction.

In an embodiment, the at least one processor may be configured to generate information representing a connection portion configured to connect the cervical prosthesis and the guide portion.

In an embodiment, the connection portion may include: a body portion; a first connection portion configured to connect the body portion to the cervical prosthesis; and a second connection portion configured to connect the body portion to the guide portion, and wherein the information representing the connection portion includes information that makes each of one or more dimensions of the first connection portion smaller than a corresponding dimension of the second connection portion.

In an embodiment, the at least one processor may be configured to: based on the three-dimensional image model, acquire a user input for information indicating multiple points corresponding to the first line; and based on the user input, determine the first line.

In an embodiment, the at least one processor may be configured to generate the information representing the cervical prosthesis, which includes information indicating an inner surface of the cervical prosthesis and an outer surface of the cervical prosthesis that are distinguished based on the first line.

In an embodiment, the at least one processor may be configured to, based on the three-dimensional data of a surface of a worn cervical portion of the specific tooth, determine information indicating an inner surface of the cervical prosthesis such that a space is formed between the inner surface of the cervical prosthesis and the surface of the worn cervical portion of the specific tooth in case that the cervical prosthesis is mounted on the specific tooth.

In an embodiment, the at least one processor may be configured to, based on the information indicating the inner surface of the cervical prosthesis and the first line, determine information indicating an outer surface of the cervical prosthesis.

In an embodiment, the at least one processor may be configured to: determine a first surface, which is an interior region of a closed curve obtained by projecting the first line in a specific direction, which is an insertion direction of the cervical prosthesis, onto a second plane perpendicular to the specific direction; and based on the first surface and the first line, determine the information indicating the outer surface of the cervical prosthesis through a predetermined transformation algorithm.

In an embodiment, the at least one processor may be configured to generate the information representing the guide portion, which includes information indicating an inner surface of the guide portion and an outer surface of the guide portion that are distinguished based on the second line.

In an embodiment, the at least one processor may be configured to, based on the three-dimensional data of a surface of the specific tooth above the worn cervical portion of the specific tooth, determine information indicating the inner surface of the guide portion.

In an embodiment, the at least one processor may be configured to: determine a second surface obtained by offsetting the inner surface of the guide portion or the surface of the specific tooth above the worn cervical portion of the specific tooth by a fourth predetermined distance in a specific direction which is an insertion direction of the cervical prosthesis; and based on the second surface, determine information indicating the outer surface of the guide portion.

According to an embodiment of the present disclosure, a method for processing information about cervical prosthesis generation, performed by an electronic device comprising at least one processor and at least one memory configured to store instructions to be executed by the at least one processor, may include: acquiring three-dimensional data of a subject's oral cavity, the three-dimensional data including three-dimensional coordinate values; based on the three-dimensional data, generating a three-dimensional image model; based on the three-dimensional image model, determining a first line included in a cervical prosthesis for a specific tooth of the subject; based on the first line, determining a second line included in a guide portion configured to assist insertion of the cervical prosthesis, based on the first line, generating information representing the cervical prosthesis; and based on the second line, generating information representing the guide portion.

According to an embodiment of the present disclosure, for a non-transitory computer-readable recording medium storing instructions executable by at least one processor, the instructions, when executed by the at least one processor, may cause the at least one processor to: acquire three-dimensional data of a subject's oral cavity, the three-dimensional data including three-dimensional coordinate values; based on the three-dimensional data, generate a three-dimensional image model; based on the three-dimensional image model, determine a first line included in a cervical prosthesis for a specific tooth of the subject; based on the first line, determine a second line included in a guide portion configured to assist insertion of the cervical prosthesis; based on the first line, generate information representing the cervical prosthesis; and based on the second line, generate information representing the guide portion.

### ADVANTAGEOUS EFFECTS

According to the present disclosure, it is possible to facilitate attachment of a prosthesis to a worn cervical portion.

According to the present disclosure, it is possible to manufacture a prosthesis having an appropriate shape to be inserted into a worn cervical portion.

According to the present disclosure, it is possible to simplify a process of generating a cervical prosthesis using a 3D printer and CAD.

The effects of the technical spirit of the present disclosure are not limited to the above-described effects, and other effects not described herein will be clearly understood by those skilled in the art from the description of the specification.

### DESCRIPTION OF DRAWINGS

FIG. 1 illustrates acquisition of an image of a patient's oral cavity by using an oral scanner according to an embodiment of the present disclosure.
FIG. 2A is a block diagram of an electronic device and an oral scanner according to an embodiment of the present disclosure. FIG. 2B is a perspective view of the oral scanner according to an embodiment of the present disclosure.
FIG. 3 illustrates a method for generating a three-dimensional image of an oral cavity according to an embodiment of the present disclosure.
FIG. 4 illustrates a first line according to an embodiment of the present disclosure.
FIG. 5 illustrates endpoints and directions according to an embodiment of the present disclosure.
FIG. 6 illustrates a first plane and first to third points according to an embodiment of the present disclosure.
FIG. 7 illustrates fourth to sixth points according to an embodiment of the present disclosure.
FIG. 8 illustrates a second line according to an embodiment of the present disclosure.
FIG. 9 illustrates a process of generating a second line according to an embodiment of the present disclosure.
FIG. 10 illustrates a process of generating a second line according to an embodiment of the present disclosure.
FIG. 11 illustrates an inner surface of a guide portion according to an embodiment of the present disclosure.
FIG. 12 illustrates an inner surface of a cervical prosthesis according to an embodiment of the present disclosure.
FIG. 13 illustrates a process of generating an inner surface of a cervical prosthesis according to an embodiment of the present disclosure.
FIG. 14 illustrates an outer surface of a guide portion according to an embodiment of the present disclosure.
FIG. 15 illustrates a process of generating an outer surface of a cervical prosthesis according to an embodiment of the present disclosure.
FIG. 16 is a diagram showing a process of generating an outer surface of a cervical prosthesis according to an embodiment of the present disclosure.
FIG. 17 illustrates a process of generating an outer surface of a cervical prosthesis according to an embodiment of the present disclosure.
FIG. 18 illustrates a cervical prosthesis and a guide portion according to an embodiment of the present disclosure.
FIG. 19 illustrates a cervical prosthesis, a guide portion, and a connection portion according to an embodiment of the present disclosure.
FIG. 20 is an operation flowchart illustrating a method for processing information about the generation of a cervical prosthesis according to an embodiment of the present disclosure.

### MODE FOR INVENTION

Embodiments of the present disclosure are illustrated for describing the technical spirit of the present disclosure. The scope of the claims according to the present disclosure is not limited to the embodiments described below or to the detailed descriptions of these embodiments.

All technical or scientific terms used in the present disclosure have meanings that are generally understood by those skilled in the art to which the present disclosure pertains, unless otherwise specified. All terms used in the present disclosure are selected for the purpose of more clear description of the present disclosure, and are not selected to limit the scope of claims according to the present disclosure.

The expressions "include," "provided with," "have," and the like used in the present disclosure should be understood as open-ended terms connoting the possibility of inclusion of other embodiments, unless otherwise mentioned in a phrase or sentence including the expressions.

The singular expressions used in the present disclosure may include plural meanings, unless otherwise mentioned, and the same applies to a singular expression used in the claims. The terms "first," "second," etc. used in the present disclosure are used to distinguish between multiple components, and are not intended to limit the order or importance of the relevant components.

The term "unit" used in the present disclosure means a software component or hardware component, such as a field-programmable gate array (FPGA) and an application specific integrated circuit (ASIC). However, a "unit" is not limited to software and hardware. The "unit" may be configured to be in an addressable storage medium or may be configured to execute one or more processors. For example, the "unit" may include components, such as software components, object-oriented software components, class components, and task components, as well as processors, functions, attributes, procedures, subroutines, segments of program codes, drivers, firmware, microcode, circuits, data, databases, data structures, tables, arrays, and variables. Functions provided in components and "unit" may be combined into a smaller number of components and "units" or further subdivided into additional components and "units."

The expression "based on" used in the present disclosure is used to describe one or more factors that affect a decision, an act or operation of decision or determination described in a phrase or sentence including the relevant expression, and this expression does not exclude additional factor that affects the action or operation of decision or determination.

When a component is described as being "coupled to" or "connected to" another component, it should be understood that the component may be coupled or connected directly to the other component or that the component may be coupled or connected to the other component via a new intervening component.

Expressions such as "A, B, and C," "A, B, or C," "A, B, and/or C," "at least one of A, B, and C," "at least one of A, B, or C," and "at least one of A, B, and/or C," used in the present disclosure, may each refer to listed items or all possible combinations of listed items. For example, "at least one of A or B" may refer to all of (1) at least one A, (2) at least one B, and (3) at least one A and at least one B.

The expression "configured to" used in the present disclosure may have meanings such as "set to," "having the capacity to," "modified to," "made to," or "capable of," depending on the context. This expression is not limited to the meaning of "specifically designed to" in hardware. For example, a processor configured to perform a specific operation may refer to a general-purpose processor capable of performing the specific operation by executing software, or may refer to a special-purpose computer structured through programming to perform the specific operation.

Hereinafter, embodiments of the present disclosure will be described with reference to the accompanying drawings. In the accompanying drawings, identical or corresponding components are denoted by the same reference numerals. In the following description of embodiments, repeated descriptions of the identical or relevant components will be omitted. However, even if a description of a component is omitted, such a component is not intended to be excluded in an embodiment.

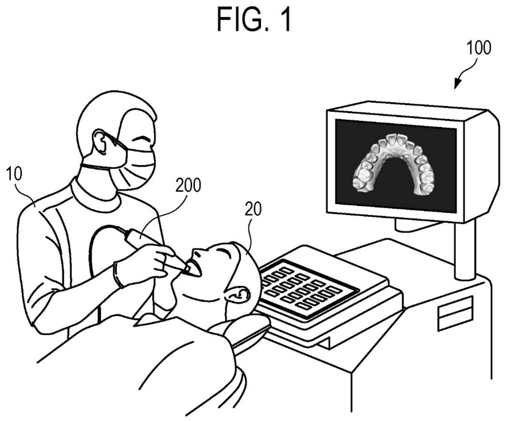

FIG. 1 illustrates acquisition of an image of a patient's oral cavity using a three-dimensional scanner 200 according to an embodiment of the present disclosure. According to an embodiment, the three-dimensional scanner 200 may be a dental medical device for acquiring an image of the interior of the oral cavity of a subject 20. For example, the three-dimensional scanner 200 may be an intraoral scanner. As illustrated in FIG. 1, a user 10 (e.g., a dentist or a dental hygienist) may use the three-dimensional scanner 200 to acquire images of the oral cavity of the subject 20 (e.g., a patient) from the subject 20. In another example, the user 10 may acquire an image of the oral cavity of the subject 20 from a diagnostic model (e.g., a plaster model or an impression model) that replicates the shape of the oral cavity of the subject 20. Hereinafter, for convenience of description, it will be described that an image of the oral cavity of the subject 20 is acquired by scanning the oral cavity of the subject 20. However, the present disclosure is not limited thereto, and it is also possible to acquire an image of another part of the subject 20 (e.g., an ear of the subject 20). The three-dimensional scanner 200 may have a shape that allows the three-dimensional scanner to be inserted into and withdrawn from the oral cavity and may be a handheld scanner that allows the user 10 to freely adjust a scanning distance and scanning angle.

The three-dimensional scanner 200 according to an embodiment may be inserted into the oral cavity of the subject 20 and scan the interior of the oral cavity in a non-contact manner to acquire an image of the oral cavity. The image of the oral cavity may include at least one tooth, gingiva, and an artificial structure insertable into the oral cavity (e.g., an orthodontic device including a bracket and a wire, an implant, a denture, and an orthodontic auxiliary tool inserted into the oral cavity). The three-dimensional scanner 200 may emit light into the oral cavity of the subject 20 (e.g., at least one tooth or gingiva of the subject 20) by using a light source (or projector), and may receive light reflected from the oral cavity of the subject 20 via a camera (or at least one image sensor). According to another embodiment, the three-dimensional scanner 200 may also acquire an image of an oral diagnostic model by scanning the oral diagnostic model. If the oral diagnostic model is a diagnostic model that replicates the shape of the oral cavity of the subject 20, the image of the oral diagnostic model may be an image of the subject's oral cavity. Hereinafter, for convenience of description, the description will be made assuming that an image of the oral cavity is acquired by scanning the interior of the oral cavity of the subject 20, but the present disclosure is not limited thereto.

The three-dimensional scanner 200 according to an embodiment may acquire a surface image of the oral cavity of the subject 20 as a two-dimensional image, based on information received via a camera. The surface image of the oral cavity of the subject 20 may include at least one tooth, gingiva, or artificial structure of the subject 20, or at least one of the cheek, tongue, or lips of the subject 20. The surface image of the oral cavity of the subject 20 may be a two-dimensional image.

The two-dimensional image of the oral cavity acquired by the three-dimensional scanner 200 according to an embodiment may be transmitted to an electronic device 100 connected via a wired or wireless communication network. The electronic device 100 may be a computer device or a portable communication device. The electronic device 100 may generate a three-dimensional image of the oral cavity (or a three-dimensional oral image, a three-dimensional oral model) that represents the oral cavity in three dimensions, based on the two-dimensional image of the oral cavity received from the three-dimensional scanner 200. The electronic device 100 may generate a three-dimensional image of the oral cavity by three-dimensionally modeling the internal structure of the oral cavity, based on the received two-dimensional image of the oral cavity.

According to another embodiment, the three-dimensional scanner 200 may scan the oral cavity of the subject 20 to acquire two-dimensional images of the oral cavity, generate a three-dimensional image of the oral cavity based on the acquired two-dimensional images of the oral cavity, and transmit the generated three-dimensional image of the oral cavity to the electronic device 100.

The electronic device 100 according to an embodiment may be communicatively connected to a cloud server (not shown). In this case, the electronic device 100 may transmit the two-dimensional images of the oral cavity of the subject 20 or the three-dimensional image of the oral cavity to the cloud server, and the cloud server may store the two-dimensional image of the oral cavity of the subject 20 or the three-dimensional image of the oral cavity received from the electronic device 100.

According to another embodiment, in addition to a handheld scanner used by being inserted into the oral cavity of the subject 20, a table scanner (not shown) that is fixed and used at a specific location may also be used as the three-dimensional scanner. The table scanner may generate a three-dimensional image of the oral diagnostic model by scanning the oral diagnostic model. In this case, since a light source (or a projector) and a camera of the table scanner are fixed, the user may scan the oral diagnostic model by moving an arm that holds the oral diagnostic model in place. Compared to handheld scanners, the table scanner has a relatively lower possibility of noise being caused by other objects intervening between the camera and the diagnostic model during scanning. However, the embodiments of the present disclosure are applicable not only to handheld scanners but also to table scanners and other types of three-dimensional scanners.

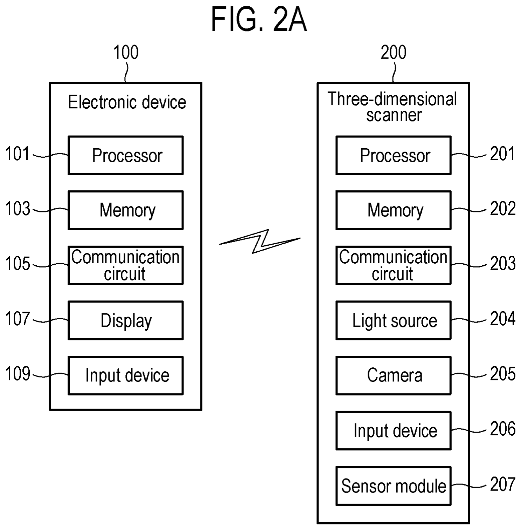

FIG. 2A is a block diagram of an electronic device 100 and a three-dimensional scanner 200 according to an embodiment of the present disclosure. The electronic device 100 and the three-dimensional scanner 200 may be communicatively connected to each other via a wired or wireless communication network, and may transmit and receive various types of data to and from each other.

The three-dimensional scanner 200 according to an embodiment may include a processor 201, a memory 202, a communication circuit 203, a light source 204, a camera 205, an input device 206, and/or a sensor module 207. At least one component included in the three-dimensional scanner 200 may be omitted, or other components may be added to the three-dimensional scanner 200. Additionally or alternatively, some components may be integrated and implemented together, or may be implemented as a single entity or multiple entities. At least some components within the three-dimensional scanner 200 may be interconnected via a bus, general-purpose input/output (GPIO), serial peripheral interface (SPI), mobile industry processor interface (MIPI), or the like to exchange data and/or signals.

The processor 201 of the three-dimensional scanner 200 according to an embodiment is an element capable of performing operation or data processing related to the control and/or communication of each component of the three-dimensional scanner 200, and may be operatively connected to the components of the three-dimensional scanner 200. The processor 201 may load commands or data received from other components of the three-dimensional scanner 200 into the memory 202, process commands or data stored in the memory 202, and store result data. The memory 202 of the three-dimensional scanner 200 according to an embodiment may store instructions related to the above-described operations of the processor 201.

According to an embodiment, the communication circuit 203 of the three-dimensional scanner 200 may establish a wired or wireless communication channel with an external device (e.g., the electronic device 100), and may transmit and receive various types of data to and from the external device. According to an embodiment, in order to communicate with the external device in a wired manner, the communication circuit 203 may include at least one port for connection to the external device via a wired cable. In this case, the communication circuit 203 may perform communication with the external device that is connected via a wire through the at least one port. According to an embodiment, the communication circuit 203 may be configured to be connected to a cellular network (e.g., 3G, LTE, 5G, WiBro, or WiMAX) by including a cellular communication module. According to an embodiment, the communication circuit 203 may include a short-range communication module to transmit and receive data to and from external devices by using short-range communication (e.g., Wi-Fi, Bluetooth, Bluetooth Low Energy (BLE), or UWB), but is not limited thereto. According to an embodiment, the communication circuit 203 may include a non-contact communication module for non-contact communication. Non-contact communication may include at least one non-contact proximity communication technology, such as near-field communication (NFC), radio frequency identification (RFID) communication, or magnetic secure transmission (MST) communication.

The light source 204 of the three-dimensional scanner 200 according to an embodiment may emit light toward the oral cavity of the subject 20. For example, the light emitted from the light source 204 may be structured light having a predetermined pattern (e.g., a stripe pattern where straight lines of different colors appear consecutively). The pattern of the structured light may be generated using, for example, a patterned mask or a digital micro-mirror device (DMD), but is not limited thereto. The camera 205 of the three-dimensional scanner 200 according to an embodiment may acquire an image of the oral cavity of the subject 20 by receiving reflected light reflected by the oral cavity of the subject 20. The camera 205 may include, for example, a left camera corresponding to a left eye's field of view and a right camera corresponding to a right eye's field of view to construct a three-dimensional image according to a photogrammetric method. The camera 205 may include at least one image sensor such as a CCD sensor or a CMOS sensor.

The input device 206 of the three-dimensional scanner 200 according to an embodiment may receive a user input for controlling the three-dimensional scanner 200. The input device 206 may include a button for receiving push operations from the user 10, a touch panel for detecting a touch of the user 10, and a voice recognition device including a microphone. For example, the user 10 may use the input device 206 to control starting or stopping of scanning.

The sensor module 207 of the three-dimensional scanner 200 according to an embodiment may detect an operating state of the three-dimensional scanner 200 or external environmental conditions (e.g., user movements) and generate an electrical signal corresponding to the detected state. The sensor module 207 may include, for example, at least one of a gyro sensor, an acceleration sensor, a gesture sensor, a proximity sensor, or an infrared sensor. The user 10 may control the start or stop of scanning by using the sensor module 207. For example, when the user 10 holds and moves the three-dimensional scanner 200, the three-dimensional scanner 200 may control the processor 201 to start a scanning operation when the angular velocity measured via the sensor module 207 exceeds a predetermined threshold.

According to an embodiment, the three-dimensional scanner 200 may receive a user input for starting scanning via the input device 206 of the three-dimensional scanner 200 or an input device 206 of the electronic device 100, or may start scanning based on processing by the processor 201 of the three-dimensional scanner 200 or a processor 201 of the electronic device 100. When a user 10 scans the interior of the oral cavity of the subject 20 by using the three-dimensional scanner 200, the three-dimensional scanner 200 may generate a two-dimensional image of the oral cavity of the subject 20 and transmit the two-dimensional image of the oral cavity of the subject 20 to the electronic device 100 in real time. The electronic device 100 may display the received two-dimensional image of the oral cavity of the subject 20 via a display. Furthermore, the electronic device 100 may generate (construct) a three-dimensional image of the oral cavity of the subject 20, based on the two-dimensional images of the oral cavity of the subject 20, and may display the three-dimensional image of the oral cavity via the display. The electronic device 100 may also display, in real time via the display, the three-dimensional image being generated.

The electronic device 100 according to an embodiment may include at least one processor 101, at least one memory 103, a communication circuit 105, a display 107, and/or an input device 109. At least one of the components included in the electronic device 100 may be omitted, or other components may be added to the electronic device 100. Additionally or alternatively, some components may be integrated and implemented, or may be implemented as a single entity or multiple entities. At least some components within the electronic device 100 may be interconnected via a bus, general-purpose input/output (GPIO), serial peripheral interface (SPI), mobile industry processor interface (MIPI), or the like to exchange data and/or signals.

According to an embodiment, at least one processor 101 of the electronic device 100 may be an element capable of performing operation or data processing related to the control and/or communication of each component (e.g., the memory 103) of the electronic device 100. The at least one processor 101 may, for example, be operatively connected to the components of the electronic device 100. The at least one processor 101 may load commands or data received from other components of the electronic device 100 into the at least one memory 103, process the commands or data stored in the at least one memory 103, and store result data.

According to an embodiment, the at least one memory 103 of the electronic device 100 may store instructions related to the operations of the at least one processor 101. The at least one memory 103 may store data received from the three-dimensional scanner 200 (e.g., the two-dimensional image of the oral cavity acquired through oral scanning).

According to an embodiment, the communication circuit 105 of the electronic device 100 may establish a wired or wireless communication channel with an external device (e.g., the three-dimensional scanner 200 or the cloud server) and transmit and receive various data to and from the external device. According to an embodiment, the communication circuit 105 may include at least one port for connection to an external device via a wired cable in order to communicate with the external device via a wired connection. In this case, the communication circuit 105 may communicate with the external device connected in a wired manner via the at least one port. According to an embodiment, the communication circuit 105 may be configured to be connected to a cellular network (e.g., 3G, LTE, 5G, WiBro, or WiMAX) by including a cellular communication module. According to an embodiment, the communication circuit 105 may include a short-range communication module to transmit and receive data to and from external devices using short-range communication (e.g., Wi-Fi, Bluetooth, Bluetooth Low Energy (BLE), or UWB), but is not limited thereto. According to an embodiment, the communication circuit 105 may include a non-contact communication module for non-contact communication. The non-contact communication may include at least one non-contact proximity communication technology, such as near-field communication (NFC) communication, radio frequency identification (RFID) communication, or magnetic secure transmission (MST) communication.

The display 107 of the electronic device 100 according to an embodiment may display various screens based on control from the processor 101. The processor 101 may display, via the display 107, a two-dimensional image of the oral cavity of the subject 20 received from the three-dimensional scanner 200 and/or a three-dimensional image of the oral cavity in which the internal structure of the oral cavity is modeled in three dimensions. For example, a specific application may be used to display the two-dimensional image and/or the three-dimensional image of the oral cavity. In this case, the user 10 may edit, store, and delete the two-dimensional image and/or the three-dimensional image of the oral cavity.

The input device 109 of the electronic device 100 according to an embodiment may receive commands or data to be used by a component (e.g., the at least one processor 101) of the electronic device 100 from outside the electronic device 100 (e.g., a user). The input device 109 may include, for example, a microphone, a mouse, or a keyboard. According to an embodiment, the input device 109 may also be implemented as a touch sensor panel that is combined with the display 107 and is capable of recognizing contact or proximity of various external objects.

Hereinafter, it can be understood that operations described as being performed by the electronic device 100 are performed by the processor 101 of the electronic device 100 described in FIG. 2A. Furthermore, it can be understood that, among the operations described below as being performed by the electronic device 100, an operation involving communication with other devices is performed by the processor 101 of the electronic device 100 described in FIG. 2A by controlling the communication circuit 105.

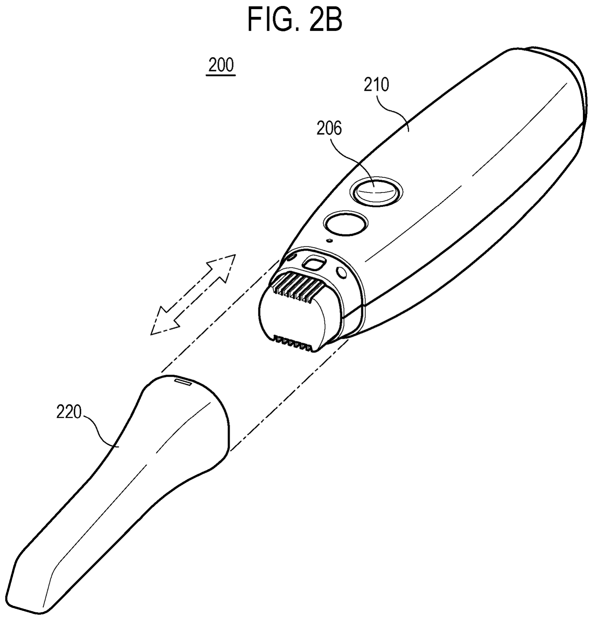

FIG. 2B is a perspective view of the three-dimensional scanner 200 according to an embodiment of the present disclosure. According to one embodiment, the three-dimensional scanner 200 may include a body 210 and a probe tip 220. The body 210 of the three-dimensional scanner 200 may be formed in a shape that is easy for the user 10 to grip by hand and use. The probe tip 220 may be formed in a shape that facilitates insertion into and withdrawal from the oral cavity of the subject 20. Further, the body 210 may be coupled to and detached from the probe tip 220. Inside the body 210, the components of the three-dimensional scanner 200 described in FIG. 2A may be arranged. An opening may be formed at one end of the body 210 so that light emitted from the light source 204 is emitted onto the subject 20 therethrough. Light emitted through the opening may be reflected by the subject 20 and reenter through the opening. The reflected light that has entered through the opening may be captured by a camera to generate an image of the subject 20. The user 10 can initiate scanning by using the input device 206 (e.g., a button) of the three-dimensional scanner 200. For example, when the user 10 touches or presses the input device 206, light may be emitted from the light source 204 onto the subject 20.

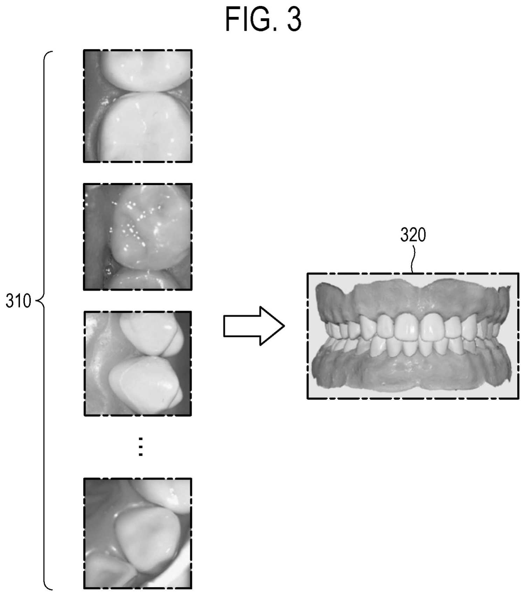

FIG. 3 illustrates a method for generating a three-dimensional image 320 of an oral cavity according to an embodiment of the present disclosure. The user 10 may scan the interior of the oral cavity of the subject 20 by moving the three-dimensional scanner 200, in which case the three-dimensional scanner 200 may acquire multiple two-dimensional images 310 of the oral cavity of the subject 20. For example, the three-dimensional scanner 200 may acquire a two-dimensional image of an area including front teeth of the subject 20, a two-dimensional image of an area including molars of the subject 20, etc. The three-dimensional scanner 200 may transmit the acquired multiple two-dimensional images 310 to the electronic device 100. According to another embodiment, the user 10 may also scan an oral diagnostic model by moving the three-dimensional scanner 200, or acquire multiple two-dimensional images of the oral diagnostic model. Hereinafter, for convenience of description, the description will be made assuming that an image of the oral cavity of the subject 20 is acquired by scanning the interior of the oral cavity of the subject 20, but the present disclosure is not limited thereto.

The electronic device 100 according to an embodiment may convert each of the multiple two-dimensional images 310 of the oral cavity of the subject 20 into a set of points having three-dimensional coordinate values. For example, the electronic device 100 may convert each of the multiple two-dimensional images 310 into a point cloud, which is a set of data points having three-dimensional coordinate values. For example, a point cloud set, which includes three-dimensional coordinate values based on the multiple two-dimensional images 310, may be stored as raw data related to the oral cavity of the subject 20. The electronic device 100 may complete an entire dental model by aligning the point cloud, which is a set of data points having three-dimensional coordinate values.

The electronic device 100 according to an embodiment may reconstruct (rebuild) a three-dimensional image of the oral cavity. For example, the electronic device 100 may use a Poisson algorithm to merge the point cloud set stored as raw data, reconstruct multiple points, and convert the multiple points into a closed three-dimensional surface, thereby reconstructing a three-dimensional image 320 of the oral cavity of the subject 20.

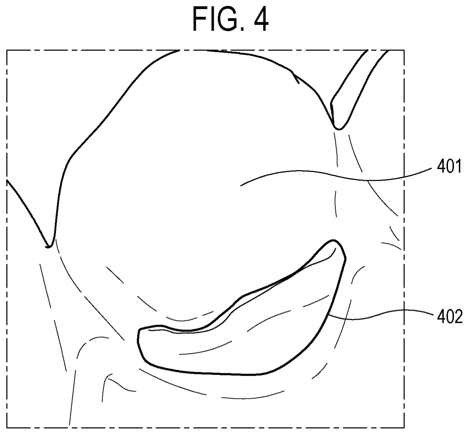

FIG. 4 illustrates a first line 402 according to an embodiment of the present disclosure. The electronic device 100 may acquire three-dimensional data including three-dimensional coordinate values of a subject's oral cavity through scanning by the three-dimensional scanner 200, and generate a three-dimensional image model based on the three-dimensional data. In another embodiment, the electronic device 100 may also acquire three-dimensional data of the oral cavity from an external device. Specifically, the electronic device 100 may download three-dimensional data of the oral cavity from an external server. Based on the three-dimensional image model, the electronic device 100 may determine the first line 402 included in a cervical prosthesis of a specific tooth of the subject. For example, the first line 402 may refer to a boundary line between a surface of a cervical prosthesis exposed to the outside and a surface of the cervical prosthesis not exposed to the outside when the cervical prosthesis is mounted to a specific tooth, or a curve that is substantially identical to that boundary line. For example, the first line 402 may be a margin line corresponding to a contour of the boundary surface between a specific tooth 401 and a cervical prosthesis.

In an embodiment, the electronic device 100 acquires a user input regarding information indicating multiple points corresponding to the first line, based on the three-dimensional image model, and may determine the first line 402, based on the user input. For example, the electronic device 100 may display the three-dimensional image model via the display 107. Subsequently, the user may input, through an input device 109, information indicating one or more specific points in the three-dimensional image model. The electronic device 100 may determine the first line 402, based on the information that is input by the user and indicates one or more specific points in the three-dimensional image model.

For example, the electronic device 100 may acquire sequential inputs of multiple specific points, wherein the first input point and the last input point entered by the user are identical. Subsequently, the electronic device 100 may determine a closed curve containing the multiple input specific points as the first line 402. Here, in order to determine the first line 402, the electronic device 100 may use any known algorithm for determining a closed curve connecting multiple ordered points on a curved surface.

In another example, the electronic device 100 may acquire an input for a specific point from a user. In this case, the electronic device 100 may determine a closed curve including a single specific point as the first line 402, based on a curvature of the surface of the specific tooth 401 in the input three-dimensional image model. Here, in order to determine the first line 402, the electronic device 100 may use any known algorithm for determining a closed curve including a single point on a curved surface.

Alternatively, the electronic device 100 may determine the first line 402, based solely on a three-dimensional image model, without relying on a user input. That is, the electronic device 100 may determine the first line 402 corresponding to the specific tooth 401, based on the three-dimensional image model, by using a known algorithm for determining a margin line corresponding to a worn cervical portion of a tooth.

In an embodiment, the electronic device 100 provides a user interface (UI) via the display 107 and the input device 109 to allow a user to modify the first line 402, and may acquire, from the user, an input instructing modification of the first line 402. For example, the input acquired from the user and instructing modification of the first line 402 may be an input based on a drag starting from a point included in the first line 402. Subsequently, the electronic device 100 may modify the first line 402, based on the input acquired from the user and instructing modification of the first line 402.

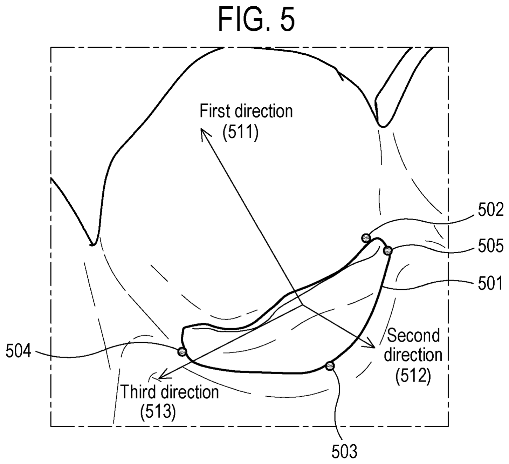

FIG. 5 illustrates endpoints and directions according to an embodiment of the present disclosure. The electronic device 100 may determine, based on a first line 501, a second line included in a guide portion that assists insertion of a cervical prosthesis. Hereinafter, the process by which the electronic device 100 determines the second line from the first line will be described with reference to FIGS. 5 to 10.

In an embodiment, the electronic device 100 may determine a first direction 511 perpendicular to the occlusal surface of a subject, based on a three-dimensional image model. The occlusal surface refers to the surface of a tooth that comes into contact and meshes with a tooth of the opposite jaw. The electronic device 100 may determine an occlusal surface of the subject corresponding to the three-dimensional image model by using an algorithm for determining the occlusal surface. Subsequently, the electronic device 100 may determine an upward direction perpendicular to the determined occlusal surface as the first direction 511.

In an embodiment, the electronic device 100 may determine a second direction 512, which is the insertion direction of a cervical prosthesis, based on the first line. Specifically, the electronic device 100 may determine the surface of a tooth that is inside the first line (i.e., the surface of a worn cervical portion). Subsequently, the electronic device 100 may determine the second direction 512, which is the insertion direction of the prosthesis, based on the sum or average of normal vectors of each unit polygon forming the surface of the tooth inside the first line. For example, the electronic device 100 may determine, as the second direction 512, the direction of a vector corresponding to the sum or average of the respective normal vectors of unit polygons that constitute the surface of the tooth inside the first line. In this case, the electronic device 100 may change the second direction 512 to the exact opposite direction if a dot product value between the determined second direction 512 and a reference vector pointing from the tooth toward the front (i.e., pointing from inside the mouth toward outside of the mouth) is negative.

Further, in an embodiment, the electronic device 100 may provide a UI via the display 107 and input device 109 to allow the user to modify the second direction 512, and may acquire, from the user, an input instructing modification of the second direction 512. For example, the input acquired from the user and instructing modification of the second direction 512 may be an input based on a drag starting from a point included in the second direction 512. Subsequently, the electronic device 100 may modify the second direction 512, based on the input acquired from the user and instructing modification of the second direction 512.

In an embodiment, the electronic device 100 may determine a third direction 513 perpendicular to both the first direction 511 and the second direction 512. For example, the electronic device 100 may determine, as the third direction 513, the direction of a vector resulting from a cross product of the second direction 512 and the first direction 511.

In an embodiment, the electronic device 100 may determine, among points included in the first line 501, an upper end point 502 and a lower end point 503, which are two points at both ends in the first direction 511, and a left end point 504 and a right end point 505, which are two end points at both ends in the third direction 504. For example, the electronic device 100 may determine, among the points included in the first line 501, the point located at the farthest end in the first direction 511 as the upper end point 502. Further, the electronic device 100 may determine, among the points included in the first line 501, the point located at the farthest end in a direction opposite to the first direction 511 as the lower end point 503. Further, the electronic device 100 may determine, among the points included in the first line 501, the point located at the farthest end in the third direction 513 as the left end point 504. Furthermore, the electronic device 100 may determine, among the points included in the first line 501, the point located at the farthest end in the third direction 513 as the right end point 505.

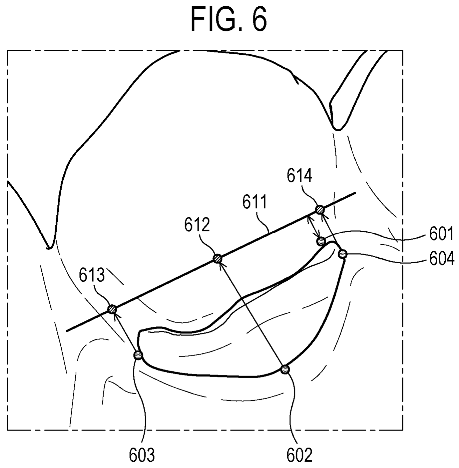

FIG. 6 illustrates a first plane 611 and first to third points according to an embodiment of the present disclosure.

In an embodiment, the electronic device 100 may determine the first plane 611 located a first predetermined distance in a first direction from an upper end point 601. Here, the first predetermined distance may be preset to a length sufficient to allow a brush to pass between a cervical prosthesis and a guide portion. Further, the first plane 611 may be a plane perpendicular to the first direction. Since the upper end point 601 is the point located at the farthest end in the first direction among the points included in the first line, the upper end point 601, a lower end point 602, a left end point 603, and a right end point 604 may all be located in the same direction (the direction opposite to the first direction) with respect to the first plane 611.

In an embodiment, the electronic device 100 may determine a first point 612, a second point 613, and a third point 614 by projecting the lower end point 602, the left end point 603, and the right end point 604 onto the first plane 611 in the first direction, respectively. In an embodiment, the electronic device 100 may determine a second line, based on the first point 612, the second point 613, and the third point 614. For example, the electronic device 100 may configure the first point 612, the second point 613, and the third point 614 as lower reference points of the second line, generate a closed curve including the lower reference points, and determine the second line, based on the generated closed curve.

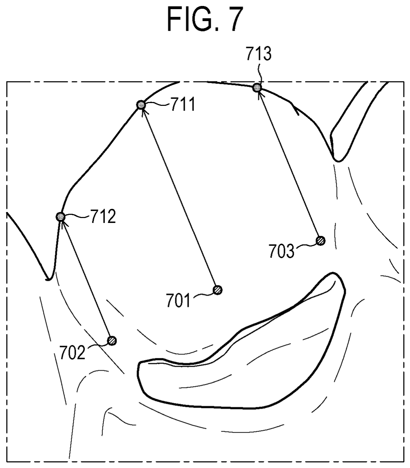

FIG. 7 illustrates fourth to sixth points according to an embodiment of the present disclosure. In an embodiment, the electronic device 100 may determine a fourth point 711, a fifth point 712, and a sixth point 713 as points where a first point 701, a second point 702, and a third point 703, when projected in a first direction, meet the upper surface of a specific tooth. Specifically, points included in the upper surface of a specific tooth may be such that rays drawn from those points in the first direction may not meet the specific tooth. In other words, the upper surface of a specific tooth may be a set of points, among points included in the surface of the specific tooth, which satisfy a condition that rays drawn from the points in the first direction do not meet the specific tooth. In an embodiment, if there are no points where the first point 701, the second point 702, and the third point 703, when projected in the first direction, meet the upper surface of a specific tooth, the electronic device 100 may output, via the display 107, a message to prompt generation of a manual guide margin line.

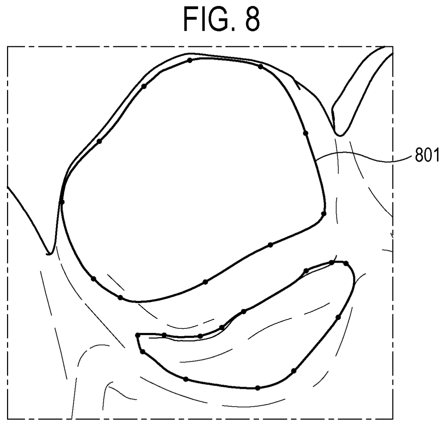

FIG. 8 illustrates a second line 801 according to an embodiment of the present disclosure. In an embodiment, the electronic device 100 may determine the second line 801, based further on at least one of the fourth point, the fifth point, and the sixth point. For example, the electronic device 100 may determine the second line 801, based on the first point, the second point, the third point, the fourth point, the fifth point, and the sixth point. Specifically, the electronic device 100 may generate a closed curve by sequentially connecting the second point 702, the first point 701, the third point 703, the sixth point 713, the fourth point 711, the fifth point 712, and the second point 702 with lines (e.g., straight lines). Subsequently, the electronic device 100 may perform smoothing processing on the generated closed curve to transform angular portions into curved-line shapes. Here, the smoothing processing may be performed using a predetermined smoothing algorithm. Subsequently, the electronic device 100 may project the smoothed closed curve onto the surface of the specific tooth. For example, the electronic device 100 may project each point included in the smoothed closed curve onto the nearest point on the surface of the specific tooth. The closed curve formed by these projected points may become the second line 801.

In an embodiment, the above-described smoothing processing and projection onto a surface of the specific tooth may each be further repeated a predetermined number of times. For example, the above-described smoothing processing and projection onto the surface of the specific tooth may each be repeated two additional times, so that each process is performed a total of three times. Through this process, the second line 801 may become a naturally smooth closed curve on the surface of the specific tooth.

In an embodiment, an internal area of a closed curve, obtained by projecting the second line 801 in the second direction onto a plane perpendicular to the second direction, which is the insertion direction of a cervical prosthesis, may be larger than an internal area of a closed curve, obtained by projecting the first line in the second direction onto the plane perpendicular to the second direction. Accordingly, when a cervical prosthesis is subsequently attached to the specific tooth, the guide portion may come into contact with the specific tooth, thereby allowing the cervical prosthesis and the guide portion to be adequately supported.

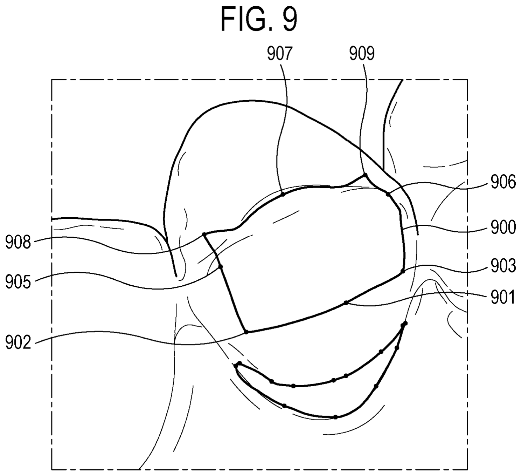

FIG. 9 illustrates a process of generating a second line according to an embodiment of the present disclosure. In another embodiment, the electronic device 100 may determine a seventh point 907, an eighth point 908, and a ninth point 909 projected onto the upper surface of a specific tooth after moving a fourth point (not shown), a fifth point 905, and a sixth point 906 by a second predetermined distance in a first direction, and then moving the fourth point, the fifth point, and the sixth point by a third predetermined distance in a direction opposite to a second direction. Here, among points included in the upper surface of the specific tooth and not located on the same straight line in the first direction as the fourth point, the fifth point 905, and the sixth point 906, there may exist a point located further in the first direction than the fourth point, the fifth point 905, and the sixth point 906, and thus the second predetermined distance may be a distance that is predetermined to move the fourth point, the fifth point 905, and the sixth point 906 further in the first direction than such a point. Therefore, the second predetermined distance may be set to a sufficiently long distance (e.g., a distance of approximately 1.5 mm to 3 mm). Additionally, the third predetermined distance may be a distance predetermined to ensure that the seventh point 907, the eighth point 908, and the ninth point 909 are not positioned too far forward on a specific tooth (i.e., to ensure that the points are positioned slightly rearward from the front surface of the specific tooth or within the center portion of the specific tooth). For example, the third predetermined distance may be predetermined to be a distance of approximately 1.5 mm to 3 mm. The electronic device 100 may determine the seventh point 907, the eighth point 908, and the ninth point 909 by projecting the points moved by the third predetermined distance onto the upper surface of the specific tooth in a direction opposite to the first direction. In another embodiment, the electronic device 100 may determine the seventh point 907, the eighth point 908, and the ninth point 909 by projecting the points moved by the third predetermined distance onto the nearest points on the upper surface of the specific tooth. Accordingly, the seventh point 907, the eighth point 908, and the ninth point 909 may be determined as points located more posteriorly on the upper surface of the specific tooth than the fourth point, the fifth point 905, and the sixth point 906.

In an embodiment, the electronic device 100 may determine the second line, based further on the seventh point 907, the eighth point 908, and the ninth point 909. For example, the electronic device 100 may determine the second line, based on a first point 901, a second point 902, a third point 903, the fifth point 905, the sixth point 906, the seventh point 907, the eighth point 908, and the ninth point 909. Specifically, the electronic device 100 may generate a closed curve 900 by sequentially connecting, with lines (e.g., straight lines), the second point 902, the first point 901, the third point 903, the sixth point 906, the ninth point 909, the seventh point 907, the eighth point 908, the fifth point 905, and the second point 902.

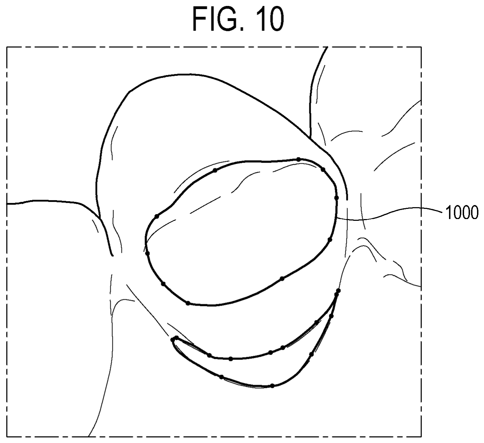

FIG. 10 illustrates a process of generating a second line according to an embodiment of the present disclosure. Following the operation described in FIG. 9, the electronic device 100 may perform smoothing processing on the generated closed curve 900 to transform angular portions into curved line forms. Here, the smoothing processing may be performed using a predetermined smoothing algorithm. Subsequently, the electronic device 100 may project the smoothed closed curve onto the surface of the specific tooth. For example, the electronic device 100 may project each point included in the smoothed closed curve onto the nearest point on the surface of the specific tooth. The closed curve formed by these projected points may be a second line 1000. In an embodiment, the above-described smoothing processing and projection onto the surface of the specific tooth may be further repeated a predetermined number of times. For example, the above-described smoothing processing and projection onto the surface of a specific tooth may be further repeated two more times, so that each process is performed a total of three times. Through this process, the second line 1000 may become a naturally smooth closed curve on the surface of the specific tooth. By generating the second line 1000 that covers even points located on a posterior portion of the upper surface of the specific tooth, a guide portion that is finally generated may also contact the posterior portion of the upper surface of the specific tooth. Consequently, the area where the guide portion contacts the specific tooth becomes larger. The guide portion may thus more stably assist the insertion of the cervical prosthesis. Furthermore, due to its L-shaped structure, the guide portion may be easily aligned with a desired portion of the specific tooth.

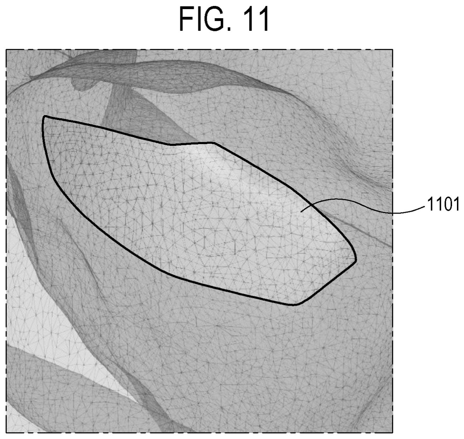

FIG. 11 illustrates an inner surface 1101 of a guide portion according to an embodiment of the present disclosure. The electronic device 100 may generate information indicating the guide portion based on a second line. Here, the information indicating the guide portion may refer to information about a set of coordinates of points constituting the volume of the guide portion or a set of coordinates of points constituting the surface of the guide portion. In an embodiment, the electronic device 100 may generate information indicating the guide portion, including information indicating the inner surface 1101 of the guide portion and the outer surface of the guide portion that are distinguished based on the second line.

In an embodiment, the electronic device 100 may determine information indicating the inner surface of the guide portion, based on scan data of the surface of a specific tooth located above a worn cervical portion of the specific tooth. For example, the electronic device 100 may determine information indicating a portion corresponding to an area included inside the second line in the scan data of the surface of the specific tooth as information indicating the inner surface of the guide portion. That is, the inner surface of the guide portion may be a curved surface having the same shape as a portion of the surface of the specific tooth.

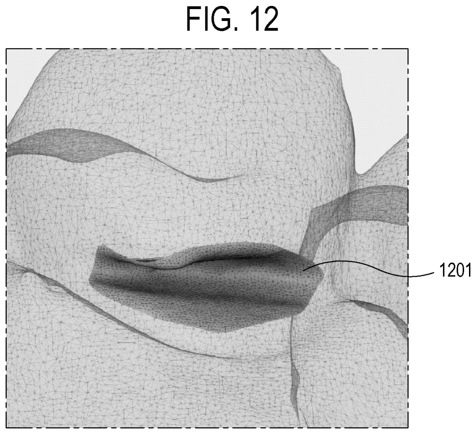

FIG. 12 illustrates an inner surface 1201 of a cervical prosthesis according to an embodiment of the present disclosure. The electronic device 100 may generate information indicating the cervical prosthesis, based on a first line. Here, the information indicating the cervical prosthesis may mean information about a set of coordinates of points constituting the volume of the cervical prosthesis or a set of coordinates of points constituting the surface of the cervical prosthesis. In an embodiment, the electronic device 100 may generate information indicating the cervical prosthesis, including information indicating the inner surface 1201 of the cervical prosthesis and the outer surface of the cervical prosthesis that are distinguished based on the first line.

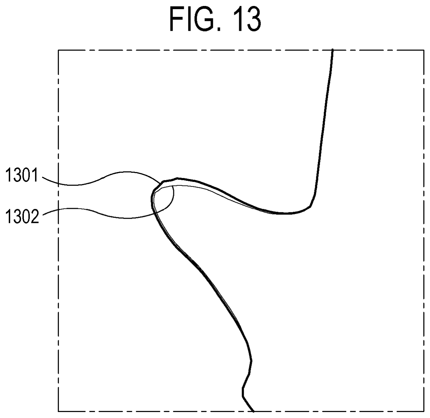

FIG. 13 illustrates a process of generating an inner surface 1302 of a cervical prosthesis according to an embodiment of the present disclosure. In an embodiment, the electronic device 100 may determine information indicating the inner surface 1302 of the cervical prosthesis, based on scan data of a surface 1301 of a worn cervical portion of a specific tooth. In an embodiment, the electronic device 100 may determine information indicating the inner surface 1302 of the cervical prosthesis such that, when the cervical prosthesis is mounted to the specific tooth, a space is formed between the inner surface 1302 of the cervical prosthesis and the surface 1301 of the worn cervical portion of the specific tooth. That is, unlike the inner surface of the guide portion, the inner surface 1302 of the cervical prosthesis may be determined to have a shape different from that of the surface 1301 of the worn cervical portion of the specific tooth. For example, the electronic device 100 may determine the inner surface 1302 of the cervical prosthesis as having a shape obtained by scaling down the shape of the surface 1301 of the worn cervical portion of the specific tooth by a specific ratio with respect to the first line. The specific ratio may be slightly less than 100% (e.g., 95%). Alternatively, in an embodiment, the specific ratio may be set such that when the cervical prosthesis is mounted to the specific tooth, a space between the inner surface 1302 of the cervical prosthesis and the surface 1301 of the worn cervical portion of the specific tooth has a predetermined specific volume. Thus, the presence of the space between the inner surface 1302 of the cervical prosthesis and the surface 1301 of the worn cervical portion of the specific tooth when the cervical prosthesis is mounted on the specific tooth allows an adhesive (e.g., cement) to perform an adhesive function in the space when the cervical prosthesis is attached to the surface 1301 of the worn cervical portion of the specific tooth. Therefore, it is undesirable for the space to be too large. In an embodiment, the electronic device 100 may acquire, from a user, an input corresponding to a specific volume, and determine a specific ratio such that the volume of the space between the inner surface of the cervical prosthesis and the surface 1301 of the worn cervical portion of the specific tooth corresponds to the acquired specific volume.

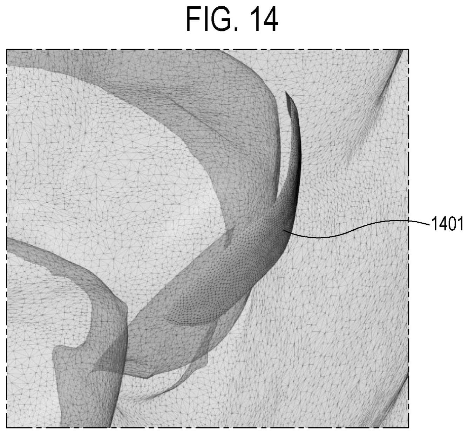

FIG. 14 illustrates the outer surface of a guide portion according to an embodiment of the present disclosure. In an embodiment, the electronic device 100 may determine a second surface 1401 obtained by offsetting, by a fourth predetermined distance in a second direction, an inner surface of a guide portion or a surface of a specific tooth above a worn cervical portion of the specific tooth. Based on the second surface 1401, the electronic device 100 may determine information indicating the outer surface 1401 of the guide portion. Here, the fourth predetermined distance may be set in advance to a specific length so that a second guide portion has an appropriate volume. For example, the fourth predetermined distance may be set to approximately 0.5 cm to 1 cm. In an embodiment, the electronic device 100 may determine an outer surface of the guide portion that includes the second surface 1401 and a surface connecting the second surface 1401 to the inner surface of the guide portion. For example, the surface connecting the second surface 1401 to the inner surface of the guide portion may be formed based on the shortest distance between the second surface 1401 and the inner surface of the guide portion, or such that the area of the surface connecting the second surface 1401 to the inner surface of the guide portion is minimized. In an embodiment, the electronic device 100 may determine the outer surface of the guide portion such that the outer surface forms a smooth curved surface by performing smoothing processing on the surface that includes the second surface 1401 and the surface connecting the second surface 1401 to the inner surface of the guide portion. Further, the electronic device 100 may determine the outer surface of the guide portion such that a second line portion, where the outer surface of the guide portion is connected to the inner surface of the guide portion, is smoothly formed as a curved surface.

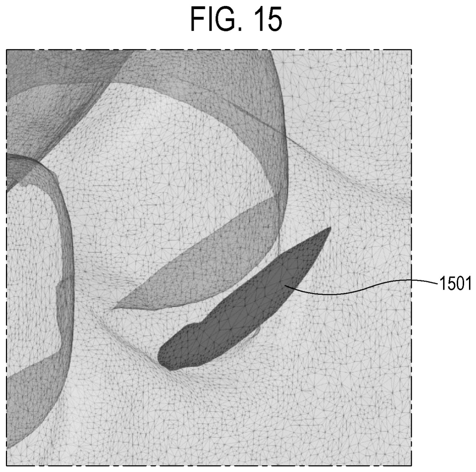

FIG. 15 illustrates a process of generating an outer surface of a cervical prosthesis according to an embodiment of the present disclosure. In an embodiment, the electronic device 100 may determine information indicating the outer surface of the cervical prosthesis, based on a first line. In an embodiment, the electronic device 100 may determine a second plane perpendicular to a second direction. For example, the electronic device 100 may determine the second plane that is perpendicular to the second direction and located at a specific distance in the second direction from a point, which is located at the farthest end in the second direction, among points included in a first line. In an embodiment, the electronic device 100 may determine a first surface 1501 that is the interior region of a closed curve formed by projecting the first line in the second direction onto the second plane perpendicular to the second direction.

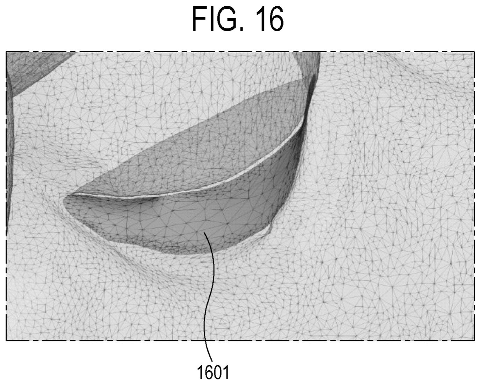

FIG. 16 illustrates a process of generating an outer surface of a cervical prosthesis according to an embodiment of the present disclosure. In an embodiment, the electronic device 100 may determine information indicating the outer surface of the cervical prosthesis, based on a first surface and a first line, by using a predetermined transformation algorithm. For example, the electronic device 100 may determine information indicating the outer surface of the prosthesis, based on the first surface and the first line, by using a radial basis function transformation (RBF transformation). The electronic device 100 may generate a curved surface 1601 by expanding the first surface through the RBF transformation so that the first surface has an area sufficient to fill the closed curve of the first line.

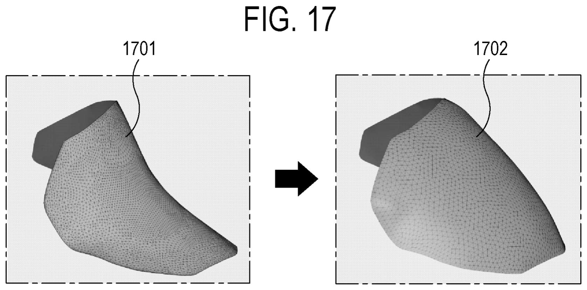

FIG. 17 illustrates a process of generating an outer surface of a cervical prosthesis according to an embodiment of the present disclosure. In an embodiment, the electronic device 100 may modify a curved surface 1701, which has been generated by expanding a first surface such that the first surface has an area sufficient to fill the closed curve of a first line, based on the curvature of a surface above a worn cervical portion of a specific tooth in the front surface of the specific tooth. Specifically, the electronic device 100 may acquire, based on a three-dimensional image model, the curvature of the surface above the worn cervical portion of the specific tooth in the front surface of the specific tooth. In relation to points included in the front face of the specific tooth, rays drawn from the points in a second direction may not meet the specific tooth. In other words, the front surface of the specific tooth may be a set of points, among points included in the surface of the specific tooth, which satisfy the condition that rays drawn from the points in the second direction does not meet the specific tooth. In an embodiment, the electronic device 100 may correct the curvature of the curved surface 1701 such that the curvature of the surface above the worn cervical portion of the specific tooth in the front surface the specific tooth and the curvature of the outer surface of the cervical prosthesis when the cervical prosthesis is mounted are continuously connected. The electronic device 100 may determine a curved surface 1702 having the corrected curvature as the outer surface of the cervical prosthesis.

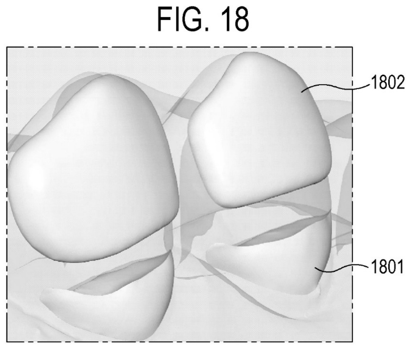

FIG. 18 illustrates a cervical prosthesis 1801 and a guide portion 1802 according to an embodiment of the present disclosure. The cervical prosthesis 1801 may be mounted on a worn cervical portion of a specific tooth. The cervical prosthesis 1801 is configured such that a space is formed between the inner surface thereof and the surface of the worn cervical portion of the specific tooth, and thus may be bonded to the surface of the worn cervical portion by using an adhesive. Further, the guide portion 1802 may be mounted on the surface of the specific tooth above the worn cervical portion of the specific tooth. The guide portion 1802 is formed such that the inner surface thereof has the same shape as a surface of the specific tooth above the worn cervical portion of the specific tooth, and thus may come into precise contact with the surface of the specific tooth above the worn cervical portion of the specific tooth.

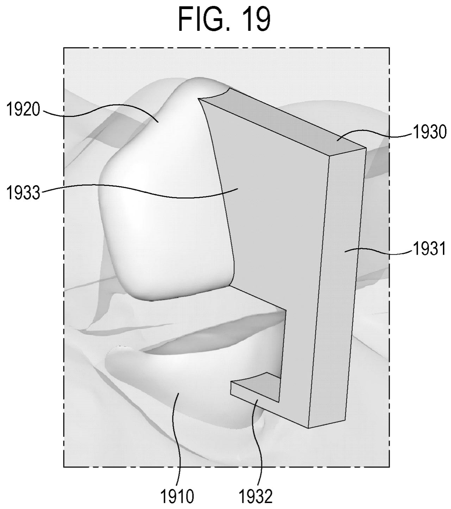

FIG. 19 illustrates a cervical prosthesis 1910, a guide portion 1920, and a connection portion 1930 according to an embodiment of the present disclosure.

In an embodiment, the electronic device 100 may generate information indicating the connection portion 1930 connecting the cervical prosthesis 1910 to the guide portion 1920. That is, the electronic device 100 may generate information representing points included within the volume of the connection portion 1930 or points included within the surface of the connection portion 1930. For example, the electronic device 100 may generate information representing the connection portion 1930 such that the connection portion is continuously connected from the outer surface of the cervical prosthesis 1910 and the outer surface of the guide portion 1920. In this case, based on the information representing the connection portion 1930 generated by the electronic device 100, the connection portion 1930 may be manufactured by a separate 3D printer. Further, based on the information representing the cervical prosthesis 1910 generated by the electronic device 100, the information for manufacturing the guide portion 1920, and the information representing the connection portion 1930, the cervical prosthesis 1910, the guide portion 1920, and the connection portion 1930 may be manufactured integrally by a separate 3D printer. In another embodiment, the information representing the cervical prosthesis 1910 and the information representing the guide portion 1920, generated by the electronic device 100, may be input into the 3D printer, and in the 3D printer, an option for automatically printing supports, which connect output objects, along with the output objects, may be selected, and the connection portion 1930 may be generated by the option.

In an embodiment, the connection portion may include a body portion 1931, a first connection portion 1932 configured to connect the body portion 1931 to the cervical prosthesis 1910, and a second connection portion 1933 configured to connect the body portion 1931 to the guide portion 1920. In an embodiment, the information representing the connection portion 1930 may include information indicating that each of one or more dimensions of the first connection portion 1932 is smaller than the corresponding dimension of the second connection portion 1933. For example, the vertical thicknesses of the first connection portion 1932 may be configured to be smaller than the vertical thicknesses of the second connection portion 1933. Alternatively, the vertical thicknesses of the first connection portion 1932 may be configured to be less than a specific ratio of the vertical thicknesses of the second connection portion 1933. The cervical prosthesis 1910, the guide portion 1920, and the connection portion 1930 may be mounted as an integrated unit onto a specific tooth. In this case, the cervical prosthesis 1910, the guide portion 1920, and the connection portion 1930, which are integrated, may be mounted on the specific tooth so that the cervical prosthesis 1910 is inserted into a worn cervical portion of the specific tooth. In this case, a portion of the cervical prosthesis 1910 facing the surface of the worn cervical portion of the specific tooth (i.e., the inner surface of the cervical prosthesis 1910) may have an adhesive applied thereto. Therefore, the cervical prosthesis 1910 and the surface of the worn cervical portion of the specific tooth may be bonded by the adhesive, and during this process, it may be necessary for the cervical prosthesis 1910 and the surface of the worn cervical portion of the specific tooth to remain fixed for a period of time, or for pressure to be applied to the cervical prosthesis 1910 in the cervical direction. The guide portion 1920, connected to the cervical prosthesis 1910 via the connection portion 1930, may contact the surface above the worn cervical portion of the specific tooth over a wide area, thereby facilitating maintaining the cervical prosthesis 1910 in a fixed state. Further, by applying pressure to the guide portion 1920 or the connection portion 1930 in the direction of the specific tooth, pressure may also be applied to the cervical prosthesis 1910 in the direction of the specific tooth. That is, it is possible to apply pressure to the cervical prosthesis 1910 more conveniently.

When bonding between the cervical prosthesis 1910 and the surface of the worn cervical portion of the specific tooth is complete, the connection portion and the guide portion are required to be removed. In this case, the first connection portion 1932 may be broken, allowing the connection portion 1930 and the guide portion 1920 to separate from the specific tooth. Here, each (e.g., vertical thickness) of one or more dimensions of the first connection portion 1932 may be manufactured smaller than the corresponding dimension of the second connection portion 1933, and thus, when pressure is applied to the connection portion 1930 in a specific direction (e.g., downward), only the first connection portion 1932 may break, while the second connection portion 1933 remains unbroken. Accordingly, the connection portion 1930 and the guide portion 1920 may be easily removed from the specific tooth. The portion where the first connection portion 1932 was connected to the cervical prosthesis 1910 may be smoothed using sandpaper or the like.

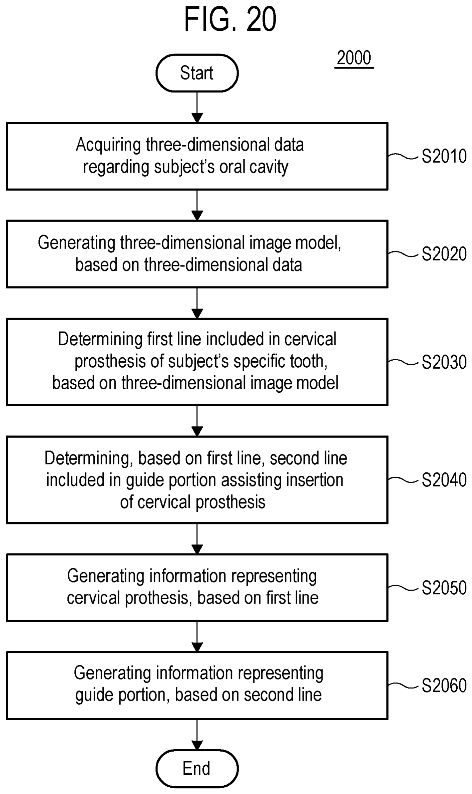

FIG. 20 is an operation flowchart illustrating a method 2000 for processing information about generating of a cervical prosthesis according to an embodiment of the present disclosure. In an embodiment, the method 2000 may include step S2010 of acquiring scan data of a subject's oral cavity through scanning by a three-dimensional scanner, step S2020 of generating a three-dimensional image model based on the scan data, step S2030 of determining, based on the three-dimensional image model, a first line included in a cervical prosthesis for a specific tooth of the subject, step S2040 of determining, based on the first line, a second line included in a guide portion assisting insertion of the cervical prosthesis, step S2050 of generating information representing the cervical prosthesis, based on the first line, and step S2060 of generating information representing the guide portion, based on the second line.

In the flowcharts of the present disclosure, the steps of the method or algorithm have been described in a sequential order. However, in addition to being performed sequentially, the steps may be performed in any order in which the steps may be arbitrarily combined. The description of the flowcharts in the present disclosure neither excludes making changes or modifications to the method or algorithm, nor implies that a certain step is necessary or desirable. In an embodiment, at least some steps may be performed in parallel, iteratively, or heuristically. In another embodiment, at least some steps may be omitted, or other steps may be added.

Embodiments according to the present disclosure may be implemented as software on a machine-readable storage medium. The software may be software for implementing the embodiments described in the present disclosure. The software may be inferred by programmers in the technical field to which the present disclosure pertains from the embodiments described in the present disclosure. For example, the software may be a program containing machine-readable commands (e.g., instructions, code, or code segments). The machine may be a device capable of operating based on commands called from the storage medium, and may be, for example, a computer. In an embodiment, the machine may be a computing device according to the embodiments described in the present disclosure. In an embodiment, the machine's processor may execute the called commands to cause the machine's components to perform functions corresponding to these commands. The storage medium may refer to any type of recording medium that stores machine-readable data. The storage medium may include, for example, ROM, RAM, CD-ROM, a magnetic tape, a floppy disk, an optical data storage device, etc. In an embodiment, the storage medium may be implemented in a distributed form, such as within computer systems connected over a network. In this case, the software may be distributed, stored, and executed in the computer systems and executed. In another embodiment, the storage medium may be a non-transitory storage medium. The non-transitory storage medium refers to a tangible medium that exists, irrespective of whether data is stored semi-permanently or temporarily, and does not include a transitory propagating signal.

Although the technical idea according to the present disclosure has been described with reference to the above embodiments, the technical spirit according to the present disclosure encompasses various substitutions, modifications, and changes that may be made without departing from the scope understandable to those skilled in the art to which the present disclosure pertains. Furthermore, it is understood that such substitutions, modifications, and changes may fall within the scope of the appended claims.

## Claims

1. An electronic device comprising:
at least one processor; and
at least one memory configured to store instructions to be executed by the at least one processor,
wherein the at least one processor is configured to, when the instructions are executed by the at least one processor:
acquire three-dimensional data of a subject's oral cavity, the three-dimensional data comprising three-dimensional coordinate values;
based on the three-dimensional data, generate a three-dimensional image model;
based on the three-dimensional image model, determine a first line included in a cervical prosthesis for a specific tooth of the subject;
based on the first line, determine a second line included in a guide portion configured to assist insertion of the cervical prosthesis;
based on the first line, generate information representing the cervical prosthesis; and
based on the second line, generate information representing the guide portion.

2. The electronic device of Claim 1, wherein an internal area of a closed curve obtained by projecting the second line in a specific direction, which is an insertion direction of the cervical prosthesis, onto a plane perpendicular to the specific direction, is larger than an internal area of a closed curve obtained by projecting the first line in the specific direction onto the plane perpendicular to the specific direction.

3. The electronic device of Claim 1, wherein the at least one processor is configured to generate information representing a connection portion configured to connect the cervical prosthesis and the guide portion.

4. The electronic device of Claim 3, wherein the connection portion comprises:
a body portion;
a first connection portion configured to connect the body portion to the cervical prosthesis; and
a second connection portion configured to connect the body portion to the guide portion, and
wherein the information representing the connection portion comprises information that makes each of one or more dimensions of the first connection portion smaller than a corresponding dimension of the second connection portion.

5. The electronic device of Claim 1, wherein the at least one processor is configured to:
based on the three-dimensional image model, acquire a user input for information indicating multiple points corresponding to the first line; and
based on the user input, determine the first line.

6. The electronic device of Claim 1, wherein the at least one processor is configured to generate the information representing the cervical prosthesis, which comprises information indicating an inner surface of the cervical prosthesis and an outer surface of the cervical prosthesis that are distinguished based on the first line.

7. The electronic device of Claim 1, wherein the at least one processor is configured to, based on the three-dimensional data of a surface of a worn cervical portion of the specific tooth, determine information indicating an inner surface of the cervical prosthesis such that a space is formed between the inner surface of the cervical prosthesis and the surface of the worn cervical portion of the specific tooth in case that the cervical prosthesis is mounted on the specific tooth.

8. The electronic device of Claim 1, wherein the at least one processor is configured to, based on information indicating an inner surface of the cervical prosthesis and the first line, determine information indicating an outer surface of the cervical prosthesis.

9. The electronic device of Claim 1, wherein the at least one processor is configured to:
determine a first surface, which is an interior region of a closed curve obtained by projecting the first line in a specific direction, which is an insertion direction of the cervical prosthesis, onto a second plane perpendicular to the specific direction; and
based on the first surface and the first line, determine information indicating an outer surface of the cervical prosthesis through a predetermined transformation algorithm.

10. The electronic device of Claim 1, wherein the at least one processor is configured to generate the information representing the guide portion, which comprises information indicating an inner surface of the guide portion and an outer surface of the guide portion that are distinguished based on the second line.

11. The electronic device of Claim 1, wherein the at least one processor is configured to, based on the three-dimensional data of a surface of the specific tooth above the worn cervical portion of the specific tooth, determine information indicating an inner surface of the guide portion.

12. The electronic device of Claim 1, wherein the at least one processor is configured to:
determine a second surface obtained by offsetting an inner surface of the guide portion or a surface of the specific tooth above the worn cervical portion of the specific tooth by a fourth predetermined distance in a specific direction which is an insertion direction of the cervical prosthesis; and
based on the second surface, determine information indicating an outer surface of the guide portion.

13. A method for processing information about cervical prosthesis generation, performed by an electronic device comprising at least one processor and at least one memory configured to store instructions to be executed by the at least one processor, the method comprising:
acquiring three-dimensional data of a subject's oral cavity, the three-dimensional data comprising three-dimensional coordinate values;
based on the three-dimensional data, generating a three-dimensional image model;
based on the three-dimensional image model, determining a first line included in a cervical prosthesis for a specific tooth of the subject;
based on the first line, determining a second line included in a guide portion configured to assist insertion of the cervical prosthesis,
based on the first line, generating information representing the cervical prosthesis; and
based on the second line, generating information representing the guide portion.

14. A non-transitory computer-readable recording medium storing instructions executable by at least one processor, the instructions, when executed by the at least one processor, causing the at least one processor to:
acquire three-dimensional data of a subject's oral cavity, the three-dimensional data comprising three-dimensional coordinate values;
based on the three-dimensional data, generate a three-dimensional image model;
based on the three-dimensional image model, determine a first line included in a cervical prosthesis for a specific tooth of the subject;
based on the first line, determine a second line included in a guide portion configured to assist insertion of the cervical prosthesis;
based on the first line, generate information representing the cervical prosthesis; and
based on the second line, generate information representing the guide portion.
